# EUROPEAN PATENT SPECIFICATION

(11) **EP 3 991 174 B1**
(45) Date of publication and mention of the grant of the patent: **09.04.2025**
(21) Application number: 20739528.6
(22) Date of filing: 19.06.2020
(51) Int. Cl.: G16H 20/10

(54) **SYSTEMS AND METHODS FOR DETECTING MISSED BOLUS DOSES**
SYSTEME UND VERFAHREN ZUR DETEKTION VON FEHLENDEN BOLUSDOSEN
SYSTÈMES ET PROCÉDÉS POUR DÉTECTER DES DOSES DE BOLUS MANQUÉES

(30) Priority: 27.06.2019 US 201962867284 P
(43) Date of publication of application: 04.05.2022
(73) Proprietor: Eli Lilly and Company, Indianapolis, IN 46285 (US)
(72) Inventor: JOHNSON, Jennal Lynn, Indianapolis, Indiana 46206-6288 (US); KATZ, Michelle Lynne, Indianapolis, Indiana 46206-6288 (US); WOLPERT, Howard Allan, Indianapolis, Indiana 46206-6288 (US)
(74) Representative: J A Kemp LLP
(86) International application number: PCT/US2020/038722
(87) International publication number: WO 2020/263706

(56) References cited:
- US-A1- 2003 163 088
- US-A1- 2003 163 088
- US-A1- 2010 094 251
- US-B1- 6 650 951
- US-B1- 6 650 951

## Description

### FIELD OF THE DISCLOSURE

The present disclosure relates to devices and methods for detecting missed insulin bolus doses. More particularly, the present disclosure relates to devices and methods for detecting when a person with diabetes may have failed to take an insulin bolus to compensate for a meal event, and to alert the person so that the person may take action to prevent or mitigate any resulting hyperglycemic excursion.

### BACKGROUND OF THE DISCLOSURE

People with diabetes generally have a diminished ability to regulate their own glucose levels. If glucose levels drop too low, people with diabetes can enter a dangerous condition called hypoglycemia. If their glucose levels rise too high, people with diabetes can enter another dangerous condition called hyperglycemia. Therefore, people with diabetes strive to keep their glucose levels within a target ideal range by dosing themselves with insulin (which lowers glucose levels) or by ingesting food and/or dosing themselves with glucagon (which raises glucose levels). Insulin can be administered in various forms, including through injections and/or using a pump. For example, insulin can be administered as a discrete dose that is injected all at once (e.g., a bolus dose), or through a steady trickle or series of micro-boluses infused using a pump over a period of multiple minutes or hours. Too much insulin can decrease glucose levels too much, sending the person into hypoglycemia. Too little insulin can leave glucose levels too high, sending the person into hyperglycemia. Therefore, people with diabetes have to dose themselves with the right amount of insulin, and at the right time.

When a person with diabetes ingests food, their glucose levels can generally be expected to rise, sometimes to dangerously high levels, unless it is compensated for by an insulin bolus. As a result, some people with diabetes require multiple daily injections (boluses) of insulin to regulate their glucose levels. These bolus injections are generally taken immediately before, concurrent with, or immediately after ingesting food. However, having to remember to take an insulin bolus every time the person eats a meal, consumes a snack, or drinks a fluid containing carbohydrates can impose a high psychological burden on the person. If a person with diabetes forgets to take an insulin bolus to counteract ingested food (e.g., misses a bolus), the person's glucose level can rise sufficiently to send the person into hyperglycemia. Extreme hyperglycemia may require immediate emergency treatment. Even cases of less extreme but chronic hyperglycemia may have negative long-term health consequences.

Therefore, a need exists for methods and devices to detect when a person with diabetes has missed a bolus. Such methods and devices should preferably notify the person with diabetes of missed boluses early enough for the person to take insulin and prevent or mitigate any hyperglycemic episode.

US 2010/094251 A1 discloses an infusion pump system that can be configured to modify alarm limit parameters as the user's insulin load increases or decreases. The infusion pump system can be configured to provide a "missed bolus" or "missed meal" alarm in response to the user's blood glucose characteristics, the user's insulin load information, or the like.

### SUMMARY

According to an exemplary embodiment of the present disclosure, a method is provided for detecting missed insulin boluses. The method comprises receiving, at a computing device, at least one first signal representative of a plurality of glucose measurements for a user and insulin dosing information for the user, the insulin dosing information comprising a time of administration for at least one insulin bolus; analyzing, with a detector of the computing device configured at a first sensitivity level, the plurality of glucose measurements and the insulin dosing information to determine whether the at least one first signal indicates the user missed an insulin bolus following a meal event; generating a user notification when the detector determines the at least one first signal indicates the user missed an insulin bolus; receiving, at the computing device, contextual information regarding the user; reconfiguring the detector, based at least on the contextual information, to detect missed boluses according to a second sensitivity level that is different from the first sensitivity level; receiving, at the computing device, at least one second signal representative of additional glucose measurements and additional insulin dosing information for the user; and analyzing, using the detector of the computing device configured at the second sensitivity level, the additional glucose measurements and the additional insulin dosing information to determine whether the at least one second signal indicates the user missed an insulin bolus following a meal event.

### BRIEF DESCRIPTION OF THE DRAWINGS

The above-mentioned and other features and advantages of this disclosure, and the manner of attaining them, will become more apparent and will be better understood by reference to the following description of embodiments of the invention taken in conjunction with the accompanying drawings, wherein:
FIG. 1 depicts a system for detecting and notifying a user of a potential missed bolus dose of insulin, according to some embodiments.
FIG. 2 depicts an exemplary process for detecting missed bolus doses of insulin, according to some embodiments.
FIG. 3 depicts an illustrative graph that shows the tradeoff between different missed bolus detection sensitivity levels, according to some embodiments.
FIGS. 4, 5, 6, 7, 8, 9, and 10 each depict an exemplary process for receiving contextual information, and reconfiguring a detector's sensitivity level, according to some embodiments.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

The term "logic," "control logic," "application," "process," "method," "algorithm," and "instructions" as used herein may include software and/or firmware executing on one or more programmable processors, application-specific integrated circuits (ASICs), field-programmable gate arrays (FPGAs), digital signal processors (DSPs), hardwired logic, or combinations thereof. Therefore, in accordance with the embodiments, various logic may be implemented in any appropriate fashion and would remain in accordance with the embodiments herein disclosed.

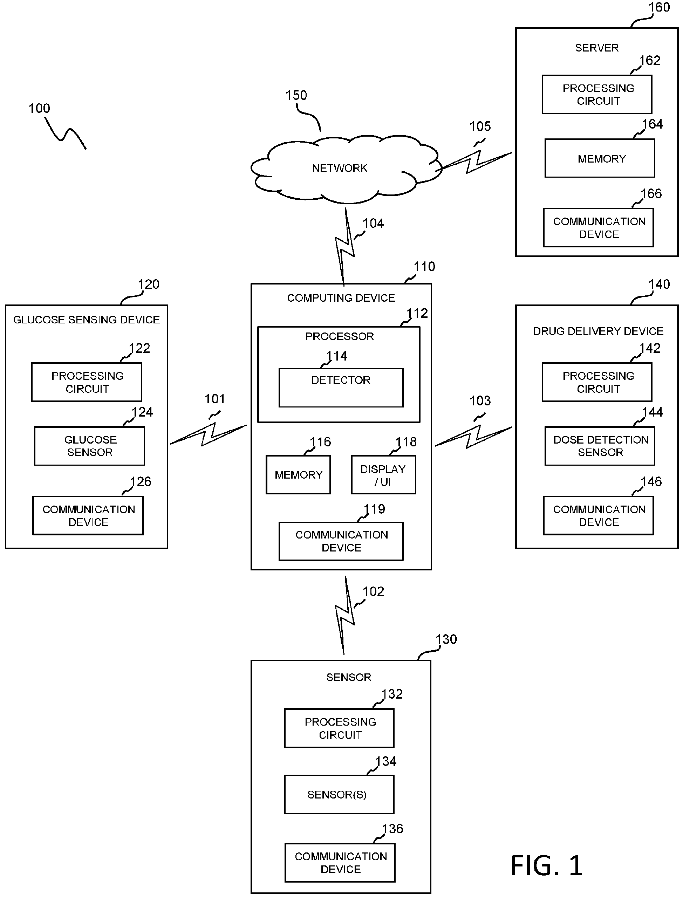

FIG. 1 depicts a system 100 for detecting and notifying a user of a potential missed bolus, according to some embodiments. System 100 includes a computing device 110 in wireless communication with a glucose sensing device 120, a drug delivery device 140, and a sensor 130. Computing device 110 may also be in communication with a server 160 via a network 150.

Computing device 110 illustratively includes a mobile device, such as a smartphone. Alternatively, any suitable computing device may be used, including but not limited to a laptop, desktop, tablet, or server computer, for example. Computing device 110 includes processor 112, memory 116, display / user-interface (UI) 118, and communication device 119.

Processor 112 includes at least one processor that executes software and/or firmware stored in memory 116 of computing device 110. The software/firmware code contains instructions that, when executed by processor 112, causes processor 112 to perform the functions described herein. Such instructions illustratively includes detector and/or detector logic 114 operative to implement the functionality described in further detail below. Memory 114 is any suitable computer readable medium that is accessible by processor 112. Memory 114 may be a single storage device or multiple storage devices, may be located internally or externally to processor 112, and may include both volatile and non-volatile media. Exemplary memory 114 includes random-access memory (RAM), read-only memory (ROM), electrically erasable programmable ROM (EEPROM), flash memory, a magnetic storage device, optical disk storage, or any other suitable medium which is configured to store data and which is accessible by processor 112.

Computing device 110 includes a display / user interface 118 in communication with processor 112 and operative to provide user input data to the system and to receive and display data, information, and prompts generated by the system. User interface 118 includes at least one input device for receiving user input and providing the user input to the system. In the illustrated embodiment, user interface 118 is a graphical user interface (GUI) including a touchscreen display operative to display data and receive user inputs. The touchscreen display allows the user to interact with presented information, menus, buttons, and other data to receive information from the system and to provide user input into the system. Alternatively, a keyboard, keypad, microphone, mouse pointer, or other suitable user input device may be provided.

Computing device 110 further includes communication device 119 that allows computing device 110 to establish wired and/or wireless communication links with other devices. Communication device 119 may comprise one or more wireless antennas and/or signal processing circuits for sending and receiving wireless communications, and/or one or more ports for receiving physical wires for sending and receiving data. Using communication device 119, computing device 110 may establish one or more short-range communication links, including one or more of communication link 101 with glucose sensing device 120, communication link 102 with sensor 130, and communication link 103 with drug delivery device 140. Such short-range communication links may utilize any known wired or wireless communication technology or protocol, including without limitation radio frequency communications (e.g., Wi-Fi, Bluetooth, Bluetooth Low Energy (BLE), Near Field Communications (NFC), RFID, and the like), infrared transmissions, microwave transmissions, and lightwave transmissions. Such short-range communication links may be either uni-directional links (e.g., data flows solely from glucose sensor 120, sensor 130, and/or device 140 to computing device 110), or bi-directional links (e.g., data flows both ways). Communication device 119 may also allow computing device 110 to establish a long-range communication link with a server 160 via a network 150, and communication links 104 and 105. The server 160 may be located remote from computing device 110, e.g., in another building, in another city, or even in another country or continent. Network 150 may comprise any cellular or data network adapted to relay information from computing device 110 to and/or from server 160, potentially via one or more intermediate nodes or switches. Examples of suitable networks 150 include a cellular network, a metropolitan area network (MAN), a wide area network (WAN), and the Internet.

Glucose sensor 120 illustratively includes any sensor adapted to measure a glucose level of a person with diabetes, such as a blood glucose monitor (BGM), a continuous glucose monitor (CGM), and/or a flash glucose monitor (FGM). Glucose sensor 120 includes a processing circuit 122, a glucose sensor 124, and communication device 126. Processing circuit 122 may include any processing circuit that receives and processes data signals, and which outputs results in the form of one or more electrical signals as a result. Processing circuit 122 may include a processor (similar to processor 112), an Application Specific Integrated Circuit (ASIC), field-programmable gate arrays (FPGAs), digital signal processors (DSPs), hardwired logic, or combinations thereof. Glucose sensor 124 comprises any sensor capable of extracting and/or analyzing analyte (e.g., blood or interstitial fluid) from the body of the person with diabetes to measure and/or record the person's glucose levels. Communication device 126 allows glucose sensor 120 to communicate with computing device 110 via communication link 101, and to relay the measured glucose levels to computing device 110.

Sensor 130 illustratively includes any sensor configured to measure at least one of a physiological or medical parameter of the person, a geographical or physical location of the person, and movement of the person, and to communicate the measured information to computing device 110. Sensor 130 may be a wearable and/or portable sensor configured to be worn on, attached to, or carried by a person with diabetes. Examples of wearable sensors includes smartwatches (e.g., the Apple Watch^{®}, a Fitbit ^{®}, and the like), heart-rate monitors, cardiac monitors, and the like. Sensor 130 may also be an implantable sensor implanted within the person's body. In yet other embodiments, sensor 130 may be neither wearable nor implantable, but may be configured to observe the person with diabetes. For example, sensor 130 may be a pressure and/or movement sensor placed on top of, underneath, or within the person's bed, and configured to record the times at which the person sleeps on the bed, as well as information regarding the quality of the person's sleep. Sensor 130 may also comprise a camera placed in the person's home, office, car, or other location that is configured to observe the person's behavior, presence, and/or appearance. Sensor 130 includes a processing circuit 132, one or more sensor(s) 134 configured to measure the above-referenced information regarding the person, and communication device 136. Processing circuit 132 may include any of the possible types of processing circuits previously described. Communication device 136 allows sensor 130 to communicate with computing device 110 via communication link 102, and to relay the measured information to computing device 110.

Drug delivery device 140 illustratively includes any device configured to deliver a dose of insulin to a person with diabetes, to measure and/or record the time and amount of dose delivered, and to communicate this information to computing device 110. The term "insulin" refers to one or more therapeutic agents including insulins, insulin analogs such as insulin lispro or insulin glargine, and insulin derivatives. Such a device is operated in a manner generally as described herein by a patient, caregiver or healthcare professional to deliver insulin to a person. The insulin delivered by device 140 may be formulated with one or more excipients. Drug delivery device 140 may be configured as a re-usable device that may be re-filled with insulin once its store of insulin is exhausted, or may be configured as a disposable device that is designed to be discarded and replaced once its store of insulin is exhausted. Drug delivery device 140 includes processing circuit 142, dose detection sensor 144, and communication device 146. Processing circuit 142 may include any of the possible types of processing circuits previously described. Dose detection sensor 144 may include any suitable sensor for detecting and/or recording the time and amount of dose delivered. Communication device 146 allows drug delivery device 140 to communicate with computing device 110 via communication link 103.

Server 160 illustratively includes any computing device configured to receive information regarding a person with diabetes from computing device 110 via network 150, process said information, and optionally, to send responses, notifications, or instructions to computing device 110 in response to said information. Server 160 includes processing circuit 162, memory 164, and communication device 166. Processing circuit 162 may include any of the possible types of processing circuits previously described. Processing circuit 162 may execute software and/or firmware stored in memory 164 of server 160. The software/firmware code contains instructions that, when executed by processing circuit 162 to perform the functions described herein. Memory 164 may also be configured to store information regarding one or more persons with diabetes, such as biographical information and/or medical information (e.g., insulin dosing records, medical history, and the like). Information received from or sent to computing device 110 may also be stored in memory 164. Memory 164 may include any of the possible types of memory previously described. Communication device 166 allows server 160 to communicate with computing device 110 via communication link 105, network 150, and communication link 104.

In some embodiments, system 100 may be modified by omitting one or more of glucose sensing device 120, sensor 130, and drug delivery device 140. For example, instead of using a connected glucose sensing device 120 as shown, a user of system 100 may instead measure or estimate his/her own glucose levels using other methods (e.g., using a non-connected glucose sensing device, such as a BGM), and then manually input the measured glucose level and the time of measurement into computing device 110. As another example, instead of using a connected drug delivery device 140 as shown, a user of system 100 may instead manually inject him or herself using a non-connected delivery device (e.g., a syringe), and then manually input the time and amount of insulin doses taken. In some embodiments, computing device 110 may function solely based on glucose measurements and insulin dosing information, and so may not require the types of information provided by sensor 130. Even if computing device 110 does use this additional information, the user may manually input information directly into computing device 110. In further embodiments, computing device 110 may operate without being connected to any network 150 and/or server 160.

In other embodiments, system 100 may be modified by adding components. For example, server 160 may be configured as a plurality of networked servers 160 that cooperate to process information. Such a configuration of networked servers may be referred to as a "cloud" of servers that perform the functions described herein. The server(s) 160 may communicate with multiple computing devices 110 via network 150, and each computing device 110 may in turn be optionally connected with one or more glucose sensing devices 120, one or more sensors 130, and one or more drug delivery devices 140.

System 100 has been described herein as implementing detector 114 within processor 110. As described in further detail below, detector 114 is configured to detect one or more indications that the user missed an insulin bolus or needs a bolus. Detector 114 may be configured to detect such indications of missed insulin boluses using different sensitivity levels, as described herein. Detector 114 may comprise software instructions and/or logic that is stored in memory 116 and which is executed by processor 112 to implement the functionality described herein. However, detector 114 may take other forms in other embodiments. For example, the functionality performed by detector 114 may be implemented at least partially by dedicated hardware and/or firmware, such as a separate and dedicated processor and/or processing circuit. In some embodiments, the functionality performed by detector 114 may also be implemented wholly or partially on server 160, glucose sensing device 120, sensor 130, and/or drug delivery device 140.

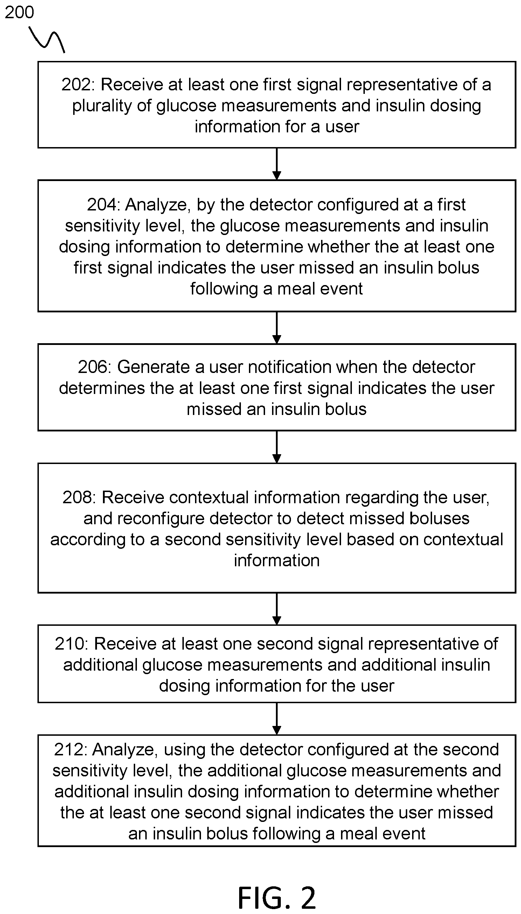

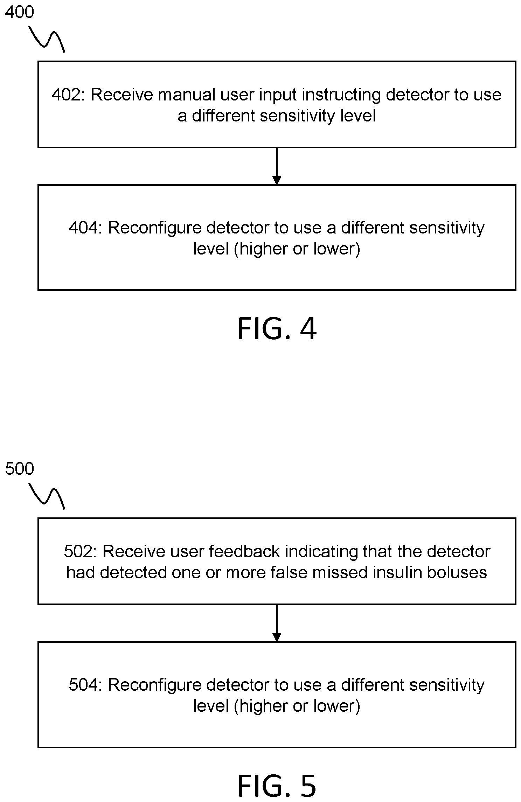

FIG. 2 depicts an exemplary process 200 for detecting missed bolus doses of insulin, according to some embodiments. For simplicity of explication, the steps depicted in FIG. 2 (and also in FIGS. 4-10) are described below as being implemented on computing device 110. However, it should be understood that any of the steps described below with respect to FIGS. 2 and 4-10 may be implemented on either computing device 110, server 160, glucose sensing device 120, sensor 130, and/or drug delivery device 140. In some embodiments, some or all of the aforementioned devices may cooperate to implement process 200, e.g., one device may perform some of the steps, while another device performs other steps, or one device may perform some of the steps with the help of intermediate computations and/or data provided by the other device.

Process 200 begins at step 202 where computing device 110 receives at least one first signal representative of a plurality of glucose measurements and insulin dosing information for a person with diabetes (herein also referred to as a "user"). The glucose measurements may be received from glucose sensing device 120 via communication link 101, or may be manually input by the user. The insulin dosing information comprises a time of administration for at least one insulin bolus taken by the user and optionally may also include the amount of insulin taken. The insulin dosing information may be received from drug delivery device 140 via communication link 103, or may be manually input by the user. After receiving this information, process 200 branches to step 204.

At step 204, computing device 110 analyzes, using a detector (e.g., detector 114), the plurality of glucose measurements and insulin dosing information to determine whether the at least one first signal indicates the user missed an insulin bolus following a meal event. As used herein, a "missed bolus" can include a situation where a user with diabetes that requires meal-time insulin has ingested food (such ingestion of food also referred to herein as a "meal event") without taking an insulin bolus to compensate for an increase in glucose levels resulting from, or expected to result from, the ingested food. A "meal event" or "food" can include any type of food, drink, or meal that can be expected to result in an increase in the user's glucose levels, including without any limitation breakfast, lunch, dinner, any snacks, and/or any drinks.

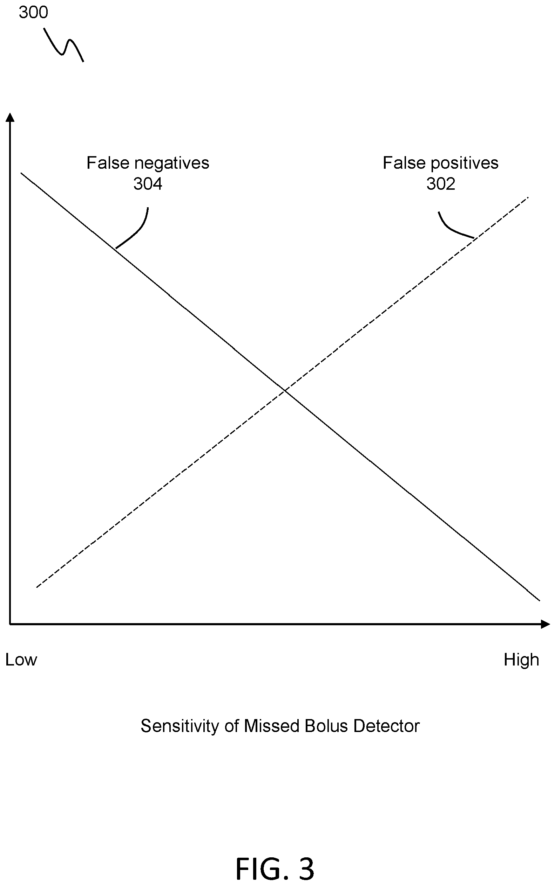

The detector may be configured to detect missed boluses according to different sensitivity levels. FIG. 3 depicts an illustrative graph that shows the tradeoff between different missed bolus detection sensitivity levels. When the detector is configured to detect missed boluses according to a low sensitivity level, the detector will output an indication of a potential missed bolus only when it is relatively confident that the user's glucose and insulin dosing information indicate the user has missed a bolus. This means the detector is likely to exhibit a relatively low false positive rate, e.g., the frequency or probability that the detector indicates the user has missed a bolus when the user has not in fact missed a bolus is likely to be relatively low. However, a low sensitivity level may also mean the detector is more likely to exhibit a relatively high false negative rate, e.g., the frequency or probability that the detector indicates the user has not missed a bolus when in fact the user has missed a bolus is likely to be relatively high. On the other hand, when the detector is configured to detect missed boluses according to a high sensitivity level, the detector will output an indication of a potential missed bolus even when it is relatively less confident that the user has missed a bolus. This means a high sensitivity detector is likely to exhibit a relatively high false positive rate and a relatively low false negative rate compared to a low sensitivity detector.

Different sensitivity levels may lead to different advantages and disadvantages. For example, a low sensitivity level detector is less likely to annoy its user with false alarms, thus mitigating alarm fatigue and the likelihood that the user will stop using the detector entirely. On the other hand, a low sensitivity level detector may fail to detect a missed bolus entirely. Even if the low sensitivity level detector detects a missed bolus, it may detect the missed bolus only after a relatively long time has passed. This means that by the time the user receives a notification regarding a missed bolus, the user's glucose levels may have already risen to undesirably high levels, and it may be too late for the user to effectively prevent or mitigate a hyperglycemic excursion.

Conversely, a high sensitivity level detector may lead to more false alarms, thus increasing the likelihood that the user will become annoyed and stop using the detector completely. However, the high sensitivity level detector will be more likely to detect a missed bolus, and/or to detect the missed bolus early enough for the user to prevent or mitigate a hyperglycemic excursion. Therefore, no one sensitivity level may be appropriate for all users or for all circumstances. The most appropriate sensitivity level to use may vary according to the user's preference, or the user's changing context, circumstances, and/or condition.

As illustrated in FIG. 3, the detector may be configured to detect missed boluses using a continuous range of sensitivity settings. Changing sensitivity settings on the detector may comprise adjusting one or more numerical thresholds in the detector's pattern-detection algorithm, or adding, modifying, or deleting one or more steps in the detector's algorithm. In some cases, changing sensitivity settings may comprise switching from one type of algorithm to a completely different type of algorithm. In some embodiments, the detector may be configured to detect missed boluses using a discrete plurality of sensitivity settings, such as two (e.g., "high" and "low"), three (e.g., "high", "medium", and "low"), four, five, or more discrete sensitivity levels. For the sake of illustration, several exemplary methods for detecting missed boluses and for adjusting the sensitivity of said methods are discussed in greater detail below.

Returning to FIG. 2, at step 206, computing device 110 generates a user notification when the detector determines the at least one first signal indicates the user missed an insulin bolus. This notification may take the form of any mechanism likely to catch the attention of the user, such as an audible tone or message, a light indicator, a pop-up message on the user-interface of computing device 110, a phone call, a text message, an email, a haptic indicator, and the like. In some embodiments, computing device 110 may also record in its memory that it sent a notification to the user (or communicate such notifications to server 106), to assist in later analysis of the frequency, timing, and/or circumstances of previous missed bolus notifications, or the user's response to missed bolus notifications. The user notification may also take the form of a notification to a user other than the user that may have missed a bolus, e.g., to friends, family, or care providers of the person with diabetes. Such a notification to some other user may take the place of, or be in addition to, the user notification to the person with diabetes. If the detector determines that the at least one first signal does not indicate the user missed an insulin bolus, computing device 110 may refrain from generating a user notification, in some embodiments.

At step 208, computing device 110 receives contextual information regarding the user, and reconfigures the detector to detect missed boluses according to a second sensitivity level (different from the first sensitivity level) based on the received contextual information. Examples of different types of contextual information are discussed in further detail below with respect to FIGS. 4-10. The second sensitivity level may be more sensitive or less sensitive than the first sensitivity level.

At step 210, computing device 110 receives at least one second signal representative of additional glucose measurements and additional insulin dosing information for the user. Such additional glucose measurements and additional insulin dosing information may be taken, recorded, and/or received later in time than the glucose measurements and insulin dosing information received at step 202.

At step 212, computing device 110 analyzes, using the detector configured at the second sensitivity level, the additional glucose measurements and the additional insulin dosing information to determine whether the at least one second signal indicates the user missed an insulin bolus following a meal event. This analysis is conducted using the second sensitivity level.

In this way, process 200 allows computing device 110 to dynamically alter the sensitivity level of its detector according to the preferences, context, circumstances, and/or condition of the user, as determined by the received contextual information. This ability to dynamically alter sensitivity levels allows process 200 to adapt to the user's preferences, context, circumstances, and/or condition, and to apply the most appropriate sensitivity level in each situation. Process 200 is therefore an improvement over previously-known missed bolus detection algorithms that use a single sensitivity level regardless of the user's preferences, context, circumstances, and/or condition.

FIG. 4 depicts an exemplary process 400 for implementing step 208 in process 200, e.g., for receiving contextual information regarding the user, and reconfiguring the detector's sensitivity level based on this contextual information, according to some embodiments. Process 400 begins at step 402, where computing device 110 receives manual user input instructing the detector to use a different sensitivity level. This manual input may instruct the user to simply increase or decrease the sensitivity level, or may specify a precise sensitivity level to switch to. At step 404, computing device reconfigures the detector to use a sensitivity level that is different from the first sensitivity level. In this way, process 400 allows a user to manually specify his/her preferences in what sensitivity level to use.

FIG. 5 depicts an exemplary process 500 for implementing step 208 in process 200, according to some embodiments. Process 500 begins at step 502, where computing device 110 receives user feedback indicating that the detector had detected one or more false missed insulin boluses. A false missed insulin bolus may include the situation where the detector had indicated that the user had missed an insulin bolus when in fact the user had not missed an insulin bolus. In other words, a false missed insulin bolus may include the situation where one or more previously-delivered missed bolus notifications were delivered in error. This feedback may be received in multiple different ways. For example, each notification of a potential missed bolus delivered in step 206 of process 200 may be accompanied by a query to the user regarding whether the notification was correct (i.e., the user had indeed missed a bolus) or incorrect (i.e., the user had not missed a bolus). The user feedback received at step 502 may comprise one or more user responses to such queries, and/or summary data derived from such user responses (e.g., "50% of the missed bolus notifications delivered in the last week were incorrect"). In some embodiments, such user feedback may be received in response to a survey question administered separate from any single missed bolus notification. The survey question may ask the user to indicate his/her perception of the missed bolus detector's accuracy, the extent to which he/she is annoyed or fatigued by excessive alarms, or may ask the user the extent to which he/she is concerned about the consequences of missing an insulin bolus. In yet other embodiments, the user feedback received at step 502 may be gathered from users other than the user currently operating computing device 110. For example, the user feedback may be received by server(s) 106 from users operating other computing devices 110. These users may, in some embodiments, be representative of the general population of users using system 100, or may be selected or filtered to be representative of the particular user currently operating computing device 110 (e.g., similar in biographical detail, medical history, geographical location, culture, and the like).

At step 504, computing device 110 reconfigures the device to use a different sensitivity level (higher or lower) based on this user feedback. For example, if the user feedback received at step 502 indicates that the user operating computing device 110 (or the community of users in communication with server(s) 106) are annoyed or fatigued by excessive alarms, computing device 110 may reconfigure the detector to use a lower sensitivity level. Conversely, if the user feedback indicates the user(s) would prefer a higher sensitivity setting (e.g., if the user(s) indicate they are concerned about potentially missing a bolus, or are more likely to miss a bolus), computing device 110 may reconfigure the detector to use a higher sensitivity level.

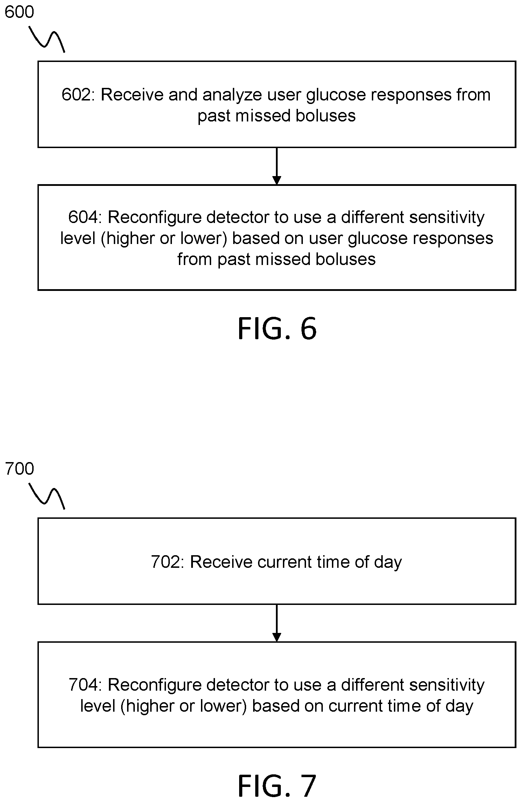

FIG. 6 depicts another exemplary process 600 for implementing step 208 in process 200, according to some embodiments. Process 600 begins at step 602, where computing device 110 receives and analyses user glucose responses from past missed boluses. These glucose responses may be gathered or recorded in response to a missed bolus notification output by the missed bolus detector, or may be gathered/recorded in response to user confirmation that he/she had missed a bolus. The glucose responses may be received from glucose sensing device 120, manually input by a user, or received via other means. These glucose responses may be analyzed to determine how severely and/or how quickly the user's glucose responded to a missed insulin bolus.

At step 604, computing device 110 reconfigures the detector to use a different sensitivity level (higher or lower) based on analysis of these glucose responses. If the user's glucose responses indicate the user's glucose levels rise by a relatively large amount, or rise relatively quickly, after a missed bolus, the health consequences of a missed bolus may be relatively severe for this particular user. Computing device 110 may accordingly reconfigure the detector to use a higher sensitivity level. Conversely, if the user's glucose responses indicate the user's glucose levels rise by a relatively small or modest amount, or tend to rise relatively slowly, the health consequences of a missed bolus may be relatively mild for this particular user. Computing device 110 may accordingly reconfigure the detector to use a lower sensitivity level to avoid unnecessarily annoying or alarming the user.

FIG. 7 depicts another exemplary process 700 for implementing step 208 in process 200, according to some embodiments. Process 700 begins at step 702, where computing device 110 receives the current time of day. The current time may be determined using an on-board clock included within or attached to computing device 110, or may be received from a source external to computing device 110 (e.g., via network 105). At step 704, computing device 110 reconfigures the detector to use a different sensitivity level (higher or lower) based on the time of day. Adjusting sensitivity level based on time of day is based on the insight that the speed and/or severity of glucose excursions due to missed boluses may differ based on time of day. For example, breakfast meals tend to be relatively high in carbohydrates with a high glycemic index, while lunch and dinner meals tend to include proteins and fats that slow absorption of carbohydrates. As a result, glucose excursions due to a missed bolus during breakfast time may be expected to result in a more severe and/or faster hyperglycemic excursion than a missed bolus during lunch time or dinner time. Accordingly, computing device 110 may configure the detector to use a higher sensitivity level during morning hours (e.g., 7-11am) and a lower sensitivity level during other hours of the day.

In some embodiments, computing device 110 and/or server 106 may analyze the timing of past glucose excursions of the patient to infer the time or times during the day at which the user is most likely to miss a bolus. For example, by analyzing a user's glucose levels over a period of weeks or months, computing device 110 and/or server 106 may determine that the user generally has no problem remembering to take a bolus for regular mealtimes, but tends to eat a sugary snack at around 3-4pm and neglect to bolus. Accordingly, computing device 110 may configure the detector to use a higher sensitivity level during the hours of 3-4pm to compensate for the user's observed habits and tendencies.

In some embodiments, process 700 may be generalized beyond reconfiguring the detector based on current time of day. For example, process 700 may be modified to reconfigure the detector based on the day of the week, or on the current date, either in place of or in addition to the current time of day. The current date may be received from an on-board calendar function included within or attached to computing device 110, or may be received from a source external to computing device 110 (e.g., via network 105). The day of the week may be relevant because the user may be more likely to forget a bolus during some days of the week compared to others. For example, a user may have relatively little difficulty remembering to take a bolus during the work week, but may tend to forget to take a bolus during the weekends - accordingly, computing device 110 may configure the detector to use a higher sensitivity level on the weekends and a lower sensitivity level during the work week. Similarly, a user may be more likely to forget to take a bolus during national holidays (e.g., Christmas) - accordingly, computing device 110 may configure the detector to use a higher sensitivity level if the current date corresponds to a national holiday, festival, or event.

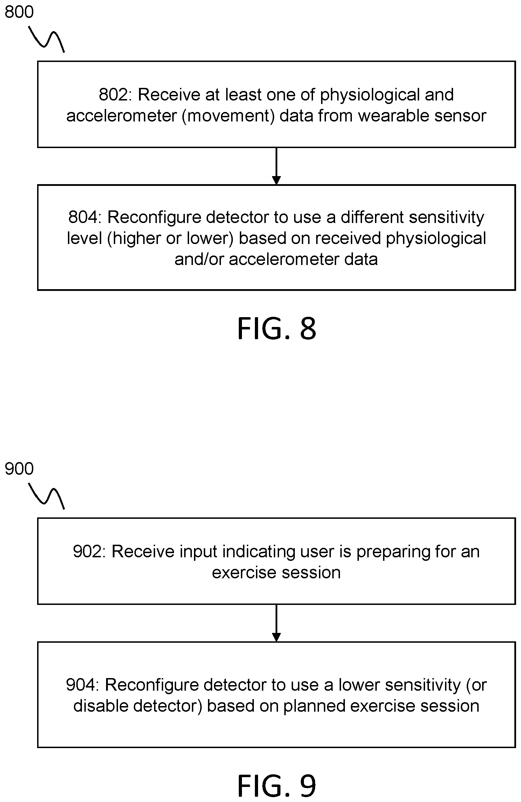

FIG. 8 depicts another exemplary process 800 for implementing step 208 in process 200, according to some embodiments. Process 800 begins at step 802, where computing device 110 receives at least one of physiological and accelerometer data pertaining to the user from sensor 130. The physiological data may be indicative of or derived from the user's heart-rate, blood pressure, blood oxygen saturation level (SpO₂), muscle oxygen saturation level (SmO₂), electrocardiogram (ECG), a breathing rate, and body temperature, while the accelerometer data may be indicative of or derived from the user's movement. Computing device 110 may draw numerous conclusions regarding the user's context, circumstances or condition based on this physiological and/or accelerometer data. For example, if the user exhibits an elevated heart-rate and/or is undergoing vigorous motion, the user may be exercising. If the user exhibits an elevated heart-rate and/or blood pressure without vigorous motion, the user may be experiencing stress. If the user exhibits an elevated temperature, the user may be ill. If the user exhibits consistent or regular motion without an elevated heart-rate and/or blood pressure, the user may be traveling or driving. If the user exhibits a low heart-rate and little to no motion, the user may be asleep.

At step 804, computing device 110 reconfigures the detector to use a different sensitivity level (higher or lower) based on the received physiological and/or accelerometer data. For example, if the user's physiological and/or accelerometer data indicates the user is exercising, computing device 110 may reconfigure the detector to use a lower sensitivity level, or even disable the detector completely. If the user's physiological data indicates the user may be ill or stressed, the detector may increase or decrease the sensitivity level as appropriate. If the user's movement / accelerometer data indicates the user may be traveling or driving, computing device 110 may reconfigure the detector to use a lower sensitivity level.

FIG. 9 depicts another exemplary process 900 for implementing step 208 in process 200, according to some embodiments. Process 900 begins at step 902, where computing device 110 receives input indicating the user is preparing for an exercise session. This input may be received by a mobile application that helps a person with diabetes safely prepare for and conduct an exercise session, while mitigating or minimizing glycemic excursions. Additional details regarding one such application are further described in the following reference: U.S. Provisional Patent No. 62/827,350, filed April 1, 2019, and entitled METHODS AND APPARATUS FOR INSULIN DOSING GUIDANCE AND DECISION SUPPORT FOR DIABETIC PATIENT EXERCISE.

While the user is preparing for an exercise session, the user may be intentionally limiting or missing his/her insulin doses because he/she anticipates that an imminent exercise will decrease his/her glucose levels. Accordingly, at step 904, computing device 110 reconfigures the detector to use a lower sensitivity level (or disable the detector entirely) if the input indicates the user is preparing for an exercise session in the near future.

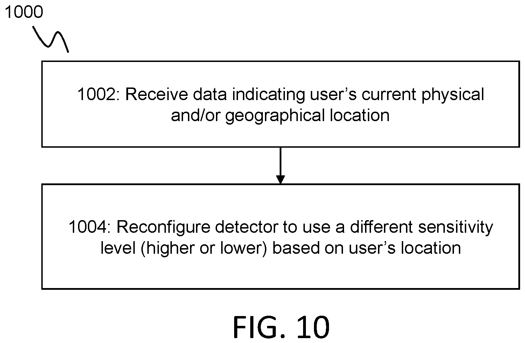

FIG. 10 depicts yet another exemplary process 1000 for implementing step 208 in process 200, according to some embodiments. Process 1000 begins at step 1002, where computing device 110 receives input indicating the user's current physical and/or geographical location. In some embodiments, the user's current physical and/or geographical location may be determined using a GPS locator installed within computing device 110, or within sensor 130. In addition or in the alternative, the user's location may be determined by detecting, using the communication device in computing device 110, wireless signals from one or more wireless access points and/or cellular nodes, and comparing the received signals, the received signal strength, and/or the received signals' directions against a database listing the locations of multiple wireless access points and/or cellular nodes.

At step 1004, computing device 110 reconfigures the detector to use a different sensitivity level (higher or lower) based on the user's location. This may be done in various ways.

For example, if the user's location corresponds to a location at which the user is likely to eat food, such as a restaurant, a cafeteria, or the user's kitchen or living room, the detector may be configured to increase the sensitivity level. In order to make this determination, computing device 110 may be configured to access a database (either stored locally on device 110 or remotely on server 106) storing information regarding different locations. The database may designate certain types of locations (e.g., restaurants, cafeterias, homes) as being areas where users are likely to ingest food, while designating other types of locations (e.g., parks / wilderness, highways, laboratories, factories) as being areas where users are less likely to ingest food. In some embodiments, the computing device may be configured to compare the user's GPS coordinates against the database to determine whether the user is likely to ingest food at the user's current location.

In some embodiments, while computing device 110 may know the coordinates of the user's current location, the computing device may not have access to contextual and/or geospatial data indicating what type of location these coordinates correspond to (e.g., what address the coordinates correspond to, or whether the coordinates correspond to a restaurant, an office building, or a user's home, etc.). This may be because the data is unavailable; the computing device 110 and/or server 106 does not have sufficient processing power or memory to store, access, or process such contextual data; or because the computing device's access to this data has been restricted to protect the user's privacy. Even in these instances, computing device 110 and/or server 106 may still adjust the detector's sensitivity level by tracking the locations at which the user has eaten food in the past. If the user's current location corresponds to a location at which the user has eaten food in the past (perhaps more than once), computing device 110 may be configured to increase the sensitivity level. In some embodiments, the device 110 and/or server 106 may also track locations at which other users have eaten food at the user's current location. If the user's current location corresponds to a location at which the user has not eaten food in the past, but at which many other users have eaten food in the past, the device 110 may still be configured to increase the detector's sensitivity level.

In yet other embodiments, computing device 110 may use the user's location to determine whether the user is in motion or traveling. For example, if the user's GPS location keeps changing, computing device 110 may infer that the user is driving, walking, or otherwise traveling. In these instances, computing device 110 may use a lower sensitivity level for the detector, as the user is less likely to be eating while in motion or traveling.

### EXEMPLARY MISSED BOLUS DETECTION METHODS

Several exemplary and illustrative methods for detecting missed boluses based on glucose measurements and insulin dosing information will now be discussed.

### I. Glucose Increase Threshold Method

One exemplary method for detecting missed boluses, referred to herein as the "Glucose Increase Threshold" method, is to determine that the user may have missed an insulin bolus if the following conditions are fulfilled:
(i) the user's glucose level increases by more than a maximum allowable glucose increase threshold (ΔGₘₐₓ, e.g., 20-60 mg/dL) within a predetermined glucose-consideration time window (T_{G}, e.g., 5-10 minutes) of the current time; and
(ii) the user has not taken an insulin bolus within a predetermined bolus-consideration time period (T_{B}, e.g., 2 hours) of the current time.

The parameters ΔGₘₐₓ, T_{G}, and T_{B} may be adjusted to make the Glucose Increase Threshold method more or less sensitive. For example, increasing the maximum allowable glucose increase threshold (ΔGₘₐₓ) would decrease the sensitivity, while decreasing ΔGₘₐₓ would increase the sensitivity. Increasing the glucose-consideration time window (T_{G}) would increase the sensitivity, while decreasing T_{G} would decrease the sensitivity. Increasing the bolus-consideration time window (T_{B}) would decrease the sensitivity, while decreasing T_{B} would increase the sensitivity.

### II. Glucose Rate-of-Change ("ROC") Threshold Method

Another exemplary method for detecting missed boluses, referred to herein as the "Glucose ROC Threshold" method, is to determine that the user may have missed an insulin bolus if the following conditions are satisfied:
(i) the user's glucose levels exhibit a rate-of-change (ROC_{G}) that is greater than a maximum allowable glucose rate-of-change threshold (ROCₘₐₓ, e.g., 2 mg/dL/hr); and
(ii) the user has not taken an insulin bolus within a predetermined bolus-consideration time period (T_{B}, e.g., 2 hours) of the current time.

ROC_{G} may be provided or calculated by some commercially available Continuous Glucose Monitors (CGM), such as the G6 CGM sensor manufactured and sold by Dexcom, Inc. For example, if a glucose sensor records three consecutive glucose readings, each spaced no more than 5 minutes apart from its closest neighbor reading in time, ROC_{G} may be calculated by dividing the difference between the last and the first glucose reading by the amount of time that has elapsed between the first and the last glucose reading. Other methods or devices for calculating ROC_{G} may also be used. Since the user remains in control of whether to act on a missed bolus notification, and no insulin is infused without the user's knowledge or approval, the methods or devices used need not be as accurate or specific as methods or devices used in wholly automated insulin infusion systems (e.g., automated insulin pumps).

The parameters ROCₘₐₓ and T_{B} may be adjusted to make the Glucose ROC Threshold method more or less sensitive. For example, increasing ROCₘₐₓ would decrease the sensitivity, while decreasing ROCₘₐₓ would increase the sensitivity. Increasing the bolus-consideration time window (T_{B}) would decrease the sensitivity, while decreasing T_{B} would increase the sensitivity.

### III. Absolute Glucose Level Threshold Method

Yet another exemplary method for detecting missed boluses, referred to herein as the "Absolute Glucose Level Threshold" method, is to determine that the user may have missed an insulin bolus if the following conditions are satisfied:
(i) the user's glucose levels exceed an absolute glucose level threshold (Gₘₐₓ, e.g., 180 mg/dL); and
(ii) the user has not taken an insulin bolus within a predetermined bolus-consideration time period (T_{B}, e.g., 2 hours)

The parameters Gₘₐₓ and T_{B} may be adjusted to make the Absolute Glucose Level Threshold method more or less sensitive. For example, increasing the maximum allowable glucose threshold (Gₘₐₓ) would decrease the sensitivity, while decreasing Gₘₐₓ would increase the sensitivity. Increasing the bolus-consideration time window (T_{B}) would decrease the sensitivity, while decreasing T_{B} would increase the sensitivity.

In some embodiments, reconfiguring the detector to detect missed boluses according to a second sensitivity level that is different from a first sensitivity level may also comprise switching between methods. For example, in some embodiments, the Glucose Increase Threshold method and the Glucose ROC Threshold Method may be considered "high" or "medium" sensitivity methods. When switching to a "low" sensitivity level, a detector may switch algorithms completely to the Absolute Glucose Level Threshold method.

The terms "first", "second", "third" and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the embodiments of the disclosure described herein are capable of operation in other sequences and/or arrangements than are described or illustrated herein.

While this invention has been described as having exemplary designs, the present invention can be further modified within the scope of this disclosure. This application is therefore intended to cover any variations, uses, or adaptations of the invention using its general principles. Further, this application is intended to cover such departures from the present disclosure as come within known or customary practice in the art to which this invention pertains.

## Claims

1. A method for detecting missed insulin boluses, comprising:
receiving, at a computing device, at least one first signal representative of a plurality of glucose measurements for a user and insulin dosing information for the user, the insulin dosing information comprising a time of administration for at least one insulin bolus;
analyzing, with a detector of the computing device configured at a first sensitivity level, the plurality of glucose measurements and the insulin dosing information to determine whether the at least one first signal indicates the user missed an insulin bolus following a meal event;
generating a user notification when the detector determines the at least one first signal indicates the user missed an insulin bolus;
**characterized in that** the method further comprises:
receiving, at the computing device, contextual information regarding the user;
reconfiguring the detector, based at least on the contextual information, to detect missed boluses according to a second sensitivity level that is different from the first sensitivity level;
receiving, at the computing device, at least one second signal representative of additional glucose measurements and additional insulin dosing information for the user; and
analyzing, using the detector of the computing device configured at the second sensitivity level, the additional glucose measurements and the additional insulin dosing information to determine whether the at least one second signal indicates the user missed an insulin bolus following a meal event.

2. The method of claim 1, wherein the detector exhibits a different false positive rate when the detector is configured according to the second sensitivity level than when the detector is configured according to the first sensitivity level.

3. The method of any of claims 1-2, wherein the contextual information comprises manual user-input instructing the detector to use a different sensitivity level than the first sensitivity level.

4. The method of any of claims 1-3, wherein the contextual information comprises manual user-input indicating the detector had detected a false missed insulin bolus.

5. The method of any of claims 1-4, wherein the contextual information comprises a current time of day.

6. The method of any of claims 1-5, wherein the contextual information comprises data measured by a wearable sensor worn by the user, wherein the data includes at least one of physiological data and accelerometer data, the physiological data optionally including at least one of a heart rate of the user, a blood pressure of the user, an electrocardiogram (ECG) of the user, a blood oxygen saturation level of the user (SpO₂), a muscle oxygen saturation level of the user (SmO₂), a breathing rate, and a body temperature of the user.

7. The method of claim 6, wherein the contextual information indicates an exercise event by the user or a sleep event by the user.

8. The method of any of claims 1-7, wherein the contextual information comprises a geographical location of the user, the contextual information optionally further comprising:
data from a database indicating whether the user has previously ingested food at the geographical location;
data from a database indicating whether other users have previously ingested food at the geographical location; and/or
data designating the user's current location as being a location where people are likely to ingest food.

9. The method of any of claims 1-8, wherein the contextual information comprises data indicating whether the user is driving.

10. The method of any of claims 1-9, wherein the plurality of glucose measurements are recorded by at least one of a blood glucose monitor (BGM), a continuous glucose monitor (CGM), and a flash glucose monitor (FGM).

11. The method of any of claims 1-10, wherein the first sensitivity level is more sensitive to missed boluses than the second sensitivity level.

12. The method of claim 11, wherein when the detector is configured to detect missed boluses according to the first sensitivity level, the detector is configured to detect missed boluses if glucose levels of the user increase by more than a maximum allowable glucose increase threshold within a predetermined glucose-consideration time window of a current time, and the user has not taken an insulin bolus within a predetermined bolus-consideration time period of the current time.

13. The method of claim 12, wherein reconfiguring the detector to detect missed boluses according to the second sensitivity level comprises:
changing at least one of the following settings: (i) increasing the maximum allowable glucose increase threshold, (ii) shortening the glucose-consideration time window, and (iii) lengthening the bolus-consideration time period; or
reconfiguring the detector to detect missed boluses if a glucose level of the user exceeds a maximum glucose threshold, and the user has not taken an insulin bolus within a second predetermined bolus-consideration time period of the current time.

14. A mobile device for detecting missed insulin boluses, the device comprising:
a communication device configured to establish one or more communication links with at least one of a glucose sensing device, a wearable sensor, and a drug delivery device;
memory storing computer-executable instructions; and
a processor configured to execute the instructions to implement the method of any of claims 1-13, wherein the communication device is optionally further configured to establish one or more communication links with a remote server via a network.

15. Non-transitory computer-readable media storing computer-executable instructions that, when executed by one or more processors, are operable to cause the one or more processors to implement the method of any of claims 1-13.

## Patentansprüche

1. Verfahren zum Erkennen versäumter Insulinboli, umfassend:
Empfangen, an einer Computervorrichtung, von mindestens einem ersten Signal, das eine Vielzahl von Glukosemessungen für einen Benutzer und Insulindosierungsinformationen für den Benutzer darstellt, wobei die Insulindosierungsinformationen einen Verabreichungszeitpunkt für mindestens einen Insulinbolus umfassen;
Analysieren, mit einem auf eine erste Empfindlichkeitsstufe konfigurierten Detektor der Computervorrichtung, der Vielzahl von Glukosemessungen und der Insulindosierungsinformationen, um zu bestimmen, ob das mindestens eine erste Signal angibt, dass der Benutzer nach einem Mahlzeitereignis einen Insulinbolus versäumt hat;
Erzeugen einer Benutzerbenachrichtigung, wenn der Detektor bestimmt, dass das mindestens eine erste Signal angibt, dass der Benutzer einen Insulinbolus versäumt hat;
**dadurch gekennzeichnet, dass** das Verfahren ferner umfasst:
Empfangen, an der Computervorrichtung, von Kontextinformationen hinsichtlich des Benutzers;
Neukonfigurieren des Detektors, basierend mindestens auf den Kontextinformationen, um versäumte Boli gemäß einer zweiten Empfindlichkeitsstufe zu erkennen, die sich von der ersten Empfindlichkeitsstufe unterscheidet;
Empfangen, an der Computervorrichtung, von mindestens einem zweiten Signal, das zusätzliche Glukosemessungen und zusätzliche Insulindosierungsinformationen für den Benutzer darstellt; und
Analysieren, unter Verwendung des auf die zweite Empfindlichkeitsstufe konfigurierten Detektors der Computervorrichtung, der zusätzlichen Glukosemessungen und der zusätzlichen Insulindosierungsinformationen, um zu bestimmen, ob das mindestens eine zweite Signal angibt, dass der Benutzer nach einem Mahlzeitereignis einen Insulinbolus versäumt hat.

2. Verfahren nach Anspruch 1, wobei der Detektor eine unterschiedliche Falsch-Positiv-Rate aufweist, wenn der Detektor gemäß der zweiten Empfindlichkeitsstufe konfiguriert ist, als wenn der Detektor gemäß der ersten Empfindlichkeitsstufe konfiguriert ist.

3. Verfahren nach einem der Ansprüche 1 bis 2, wobei die Kontextinformationen eine manuelle Benutzereingabe umfassen, die den Detektor anweist, eine unterschiedliche Empfindlichkeitsstufe als die erste Empfindlichkeitsstufe zu verwenden.

4. Verfahren nach einem der Ansprüche 1 bis 3, wobei die Kontextinformationen manuelle Benutzereingaben umfassen, die angeben, dass der Detektor einen falschen versäumten Insulinbolus erkannt hat.

5. Verfahren nach einem der Ansprüche 1 bis 4, wobei die Kontextinformationen einen aktuellen Tageszeitpunkt umfassen.

6. Verfahren nach einem der Ansprüche 1 bis 5, wobei die Kontextinformationen Daten umfassen, die durch einen tragbaren Sensor gemessen werden, der durch den Benutzer getragen wird, wobei die Daten mindestens eines von physiologischen Daten und Beschleunigungsdaten umfassen, wobei die physiologischen Daten optional mindestens eines von einer Herzfrequenz des Benutzers, einem Blutdruck des Benutzers, einem Elektrokardiogramm (EKG) des Benutzers, einem Blutsauerstoffsättigungsgrad des Benutzers (SpO₂), einem Muskelsauerstoffsättigungsgrad des Benutzers (SmO₂), einer Atemfrequenz und einer Körpertemperatur des Benutzers umfassen.

7. Verfahren nach Anspruch 6, wobei die Kontextinformationen ein Trainingsereignis durch den Benutzer oder ein Schlafereignis durch den Benutzer angeben.

8. Verfahren nach einem der Ansprüche 1 bis 7, wobei die Kontextinformationen einen geografischen Standort des Benutzers umfassen, wobei die Kontextinformationen optional ferner umfassen:
Daten aus einer Datenbank, die angeben, ob der Benutzer an dem geografischen Standort zuvor Nahrung zu sich genommen hat;
Daten aus einer Datenbank, die angeben, ob andere Benutzer an dem geografischen Standort zuvor Nahrung zu sich genommen haben; und/oder
Daten, die den aktuellen Standort des Benutzers als einen Standort kennzeichnen, an dem Menschen wahrscheinlich Nahrung zu sich nehmen.

9. Verfahren nach einem der Ansprüche 1 bis 8, wobei die Kontextinformationen Daten umfassen, die angeben, ob der Benutzer fährt.

10. Verfahren nach einem der Ansprüche 1 bis 9, wobei die Vielzahl der Glukosemessungen durch mindestens eines von einem Blutzuckermessgerät (BGM), einem kontinuierlichen Glukosemessgerät (CGM) und einem Flash-Glukosemessgerät (FGM) aufgezeichnet werden.

11. Verfahren nach einem der Ansprüche 1 bis 10, wobei die erste Empfindlichkeitsstufe empfindlicher auf verpasste Boli als die zweite Empfindlichkeitsstufe reagiert.

12. Verfahren nach Anspruch 11, wobei, wenn der Detektor konfiguriert ist, um versäumte Boli entsprechend der ersten Empfindlichkeitsstufe zu erkennen, der Detektor konfiguriert ist, um versäumte Boli zu erkennen, falls Glukosespiegel des Benutzers sich innerhalb eines vorbestimmten Glukosebeobachtungszeitfensters eines aktuellen Zeitpunkts um mehr als einen maximal zulässigen Glukoseerhöhungsschwellenwert erhöhen und der Benutzer innerhalb eines vorbestimmten Bolusbeobachtungszeitraums des aktuellen Zeitpunkts keinen Insulinbolus eingenommen hat.

13. Verfahren nach Anspruch 12, wobei das Neukonfigurieren des Detektors, um versäumte Boli gemäß der zweiten Empfindlichkeitsstufe zu erkennen, umfasst:
Ändern mindestens einer der folgenden Einstellungen: (i) Erhöhen des maximal zulässigen Glukoseerhöhungsschwellenwerts, (ii) Verkürzen des Glukosebeobachtungszeitfensters und (iii) Verlängern des Bolusbeobachtungszeitraums; oder
Neukonfigurieren des Detektors, um versäumte Boli zu erkennen, falls ein Glukosespiegel des Benutzers einen maximalen Glukoseschwellenwert überschreitet und der Benutzer innerhalb eines zweiten vorbestimmten Bolusbeobachtungszeitraums des aktuellen Zeitpunkts keinen Insulinbolus eingenommen hat.

14. Mobile Vorrichtung zum Erkennen versäumter Insulinboli, wobei die Vorrichtung umfasst:
eine Kommunikationsvorrichtung, die konfiguriert ist, um eine oder mehrere Kommunikationsverbindungen mit mindestens einen von einer Glukosesensorvorrichtung, einem tragbaren Sensor und einer Arzneimittelverabreichungsvorrichtung einzurichten;
Speicher, der computerausführbare Anweisungen speichert; und
einen Prozessor, der konfiguriert ist, um die Anweisungen auszuführen, um das Verfahren nach einem der Ansprüche 1 bis 13 zu implementieren, wobei die Kommunikationsvorrichtung optional ferner konfiguriert ist, um eine oder mehrere Kommunikationsverbindungen mit einem Remote-Server über ein Netzwerk einzurichten.

15. Nichtflüchtige, computerlesbare Medien, die computerausführbare Anweisungen speichern, die, wenn sie durch einen oder mehrere Prozessoren ausgeführt werden, betriebsfähig sind, um zu veranlassen, dass der eine oder die mehreren Prozessoren das Verfahren nach einem der Ansprüche 1 bis 13 implementieren.

## Revendications

1. Procédé de détection de bolus d'insuline manqués, comprenant :
la réception, sur un dispositif informatique, d'au moins un premier signal représentatif d'une pluralité de mesures de glucose pour un utilisateur et des informations sur le dosage d'insuline pour l'utilisateur, les informations sur le dosage d'insuline comprenant une heure d'administration d'au moins un bolus d'insuline ;
l'analyse, à l'aide d'un détecteur du dispositif informatique configuré à un premier niveau de sensibilité, de la pluralité de mesures de glucose et des informations sur le dosage d'insuline afin de déterminer si l'au moins un premier signal indique ou non que l'utilisateur a manqué un bolus d'insuline à la suite d'un repas ;
la génération d'une notification à l'utilisateur lorsque le détecteur détermine que l'au moins un premier signal indique que l'utilisateur a manqué un bolus d'insuline ;
**caractérisé en ce que** le dispositif comprend en outre :
la réception, sur le dispositif informatique, d'informations contextuelles concernant l'utilisateur ;
la reconfiguration du détecteur, sur la base au moins des informations contextuelles, afin de détecter les bolus manqués selon un second niveau de sensibilité différent du premier niveau de sensibilité ;
la réception, sur le dispositif informatique, d'au moins un second signal représentatif de mesures supplémentaires de glucose et d'informations supplémentaires sur le dosage d'insuline pour l'utilisateur ; et
l'analyse, à l'aide du détecteur du dispositif informatique configuré au second niveau de sensibilité, des mesures de glucose supplémentaires et des informations supplémentaires sur le dosage d'insuline afin de déterminer si l'au moins un second signal indique ou non que l'utilisateur a manqué un bolus d'insuline à la suite d'un repas.

2. Procédé de la revendication 1, dans lequel le détecteur présente un taux de faux positifs lorsque le détecteur est configuré selon le second niveau de sensibilité différent de celui lorsque le détecteur est configuré selon le premier niveau de sensibilité.

3. Procédé selon l'une quelconque des revendications 1 à 2, dans lequel les informations contextuelles comprennent une entrée d'utilisateur manuelle demandant au détecteur d'utiliser un niveau de sensibilité différent du premier niveau de sensibilité.

4. Procédé selon l'une quelconque des revendications 1 à 3, dans lequel les informations contextuelles comprennent une entrée d'utilisateur manuelle indiquant que le détecteur a détecté un faux bolus d'insuline manqué.

5. Procédé selon l'une quelconque des revendications 1 à 4, dans lequel les informations contextuelles comprennent une heure actuelle de la journée.

6. Procédé selon l'une quelconque des revendications 1 à 5, dans lequel les informations contextuelles comprennent des données mesurées par un capteur portable porté par l'utilisateur, les données comprenant au moins une donnée parmi les données physiologiques et les données d'accéléromètre, les données physiologiques comprenant éventuellement au moins une donnée parmi une fréquence cardiaque de l'utilisateur, une tension artérielle de l'utilisateur, un électrocardiogramme (ECG) de l'utilisateur, un niveau de saturation en oxygène du sang de l'utilisateur (SpO₂), un niveau de saturation en oxygène des muscles de l'utilisateur (SmO₂), une fréquence respiratoire et une température corporelle de l'utilisateur.

7. Procédé selon la revendication 6, dans lequel les informations contextuelles indiquent une période d'exercice physique de l'utilisateur ou une période de sommeil de l'utilisateur.

8. Procédé selon l'une quelconque des revendications 1 à 7, dans lequel les informations contextuelles comprennent une localisation géographique de l'utilisateur, les informations contextuelles comprenant en outre, éventuellement :
des données provenant d'une base de données indiquant si l'utilisateur a déjà ingéré précédemment ou non de la nourriture au niveau de la localisation géographique ;
des données provenant d'une base de données indiquant si d'autres utilisateurs ont déjà ingéré précédemment ou non de la nourriture au niveau de la localisation géographique ; et/ou
des données désignant la localisation actuelle de l'utilisateur comme une localisation où des gens sont susceptibles d'ingérer de la nourriture.

9. Procédé selon l'une quelconque des revendications 1 à 8, dans lequel les informations contextuelles comprennent des données indiquant que l'utilisateur conduit ou non.

10. Procédé selon l'une quelconque des revendications 1 à 9, dans lequel la pluralité de mesures de glucose est enregistrée par au moins un lecteur parmi un lecteur de glycémie (BGM), un lecteur de glucose en continu (CGM) et un lecteur de glucose flash (FGM).

11. Procédé selon l'une quelconque des revendications 1 à 10, dans lequel le premier niveau de sensibilité est plus sensible aux bolus manqués que le second niveau de sensibilité.

12. Procédé selon la revendication 11, dans lequel, lorsque le détecteur est configuré pour détecter des bolus manqués selon le premier niveau de sensibilité, le détecteur est configuré pour détecter des bolus manqués si d niveaux de glucose de l'utilisateur augmentent de plus d'un seuil d'augmentation maximal admissible du glucose dans une fenêtre temporelle prédéterminée de prise en compte du glucose à une heure donnée et que l'utilisateur n'a pas pris de bolus d'insuline dans une période temporelle prédéterminée de prise en compte du bolus à l'heure donnée.

13. Procédé selon la revendication 12, dans lequel la reconfiguration du détecteur pour la détection de bolus manqués selon le second niveau de sensibilité comprend :
la modification d'au moins l'un des paramètres suivants : (i) augmentation du seuil d'augmentation maximal admissible du glucose, (ii) raccourcissement de la fenêtre temporelle de prise en compte du glucose et (iii) allongement de la période de prise en compte du bolus ; ou
la reconfiguration du détecteur afin qu'il détecte des bolus manqués si le niveau de glucose de l'utilisateur dépasse un seuil maximal de glucose et que l'utilisateur n'a pas pris de bolus d'insuline dans une seconde période de temps prédéterminée de prise en compte du bolus à l'heure donnée.

14. Dispositif mobile destiné à détecter des bolus d'insuline manqués, le dispositif comprenant :
un dispositif de communication configuré pour établir une ou plusieurs liaisons de communication avec au moins un dispositif parmi un dispositif de détection de glucose, un capteur portable et un dispositif d'administration de médicaments ;
une mémoire stockant des instructions exécutables par ordinateur ; et
un processeur configuré pour exécuter les instructions afin de mettre en œuvre le procédé selon l'une quelconque des revendications 1 à 13, le dispositif de communication étant en outre configuré pour établir une ou plusieurs liaisons de communication avec un serveur distant par l'intermédiaire d'un réseau.

15. Support non transitoire lisible par ordinateur stockant des instructions exécutables par ordinateur qui, lorsqu'elles sont exécutées par un ou plusieurs processeurs, permettent d'amener le ou les processeurs à mettre en œuvre le procédé selon l'une quelconque des revendications 1 à 13.
